# FASCICULE DE BREVET EUROPEEN

(11) **EP 4 398 720 B1**
(45) Date de publication et mention de la délivrance du brevet: **01.10.2025**
(21) Numéro de dépôt: 22776968.4
(22) Date de dépôt: 22.08.2022
(51) Int. Cl.: A01K 67/36

(54) **CAGE POUR LA PRODUCTION À GRANDE ÉCHELLE D'OEUFS D'INSECTES DÉFINISSANT UN ESPACE CONFINÉ PARALLÉLÉPIPÉDIQUE POUR LES INSECTES**
KÄFIG FÜR DIE MASSENPRODUKTION VON INSEKTENEIERN MIT EINEM GESCHLOSSENEN UND QUADERFÖRMIGEN RAUM FÜR INSEKTEN
CAGE FOR LARGE SCALE PRODUCTION OF INSECT EGGS WITH A CONFINED PARALLELEPIPEDIC SPACE FOR INSECTS

(30) Priorité: 06.09.2021 FR 2109315
(43) Date de publication de la demande: 17.07.2024
(73) Titulaire: Innovafeed, 80190 Nesle (FR)
(72) Inventeur: OGGERI, Bastien, 80190 NESLE (FR); DAUSSIN, Xavier, 80190 NESLE (FR); KRAPF, Gustavo, 80190 NESLE (FR); SCHUSTER, Géraldine, NESLE 80190 (FR); MERNIER, Emmanuel, 80190 NESLE (FR); VIDAL, Olivier, 80190 NESLE (FR)
(74) Mandataire: IP Trust
(86) Numéro de dépôt international: PCT/FR2022/051596
(87) Numéro de publication internationale: WO 2023/031536

(56) Documents cités:
- FR-A1- 3 027 488
- US-B2- 10 667 502

## Description

### Domaine de l'invention

La présente invention concerne le domaine de l'élevage à grande échelle d'arthropodes et notamment d'insectes, en particulier de la mouche soldat noir, destiné par exemple à la production, à partir des larves d'insectes, d'aliments pour animaux ou humains. Elle concerne plus particulièrement les équipements destinés à l'étape de reproduction et de ponte des insectes (en anglais « mating »), débutant par l'introduction de pupes et s'achevant par la collecte des œufs qui sont ensuite transférés vers un autre équipement destiné à l'étape d'éclosion.

Cette étape de reproduction et de ponte se fait généralement à l'intérieur de cages permettant aux insectes de disposer d'un espace vital suffisant tout en restant confinés dans un espace clos. Les conditions climatiques sont contrôlées à l'intérieur de ces cages afin de garantir des conditions optimales.

### État de la technique

Le brevet FR3027488 décrit une unité d'élevage d'insectes caractérisée en ce qu'elle comprend au moins une enceinte close et un système de contrôle des conditions environnementales de l'enceinte. Ladite enceinte close comprend en outre au moins un compartiment supérieur destiné à contenir des insectes, ledit compartiment supérieur comprenant au moins un sas, généralement utilisé pour passer la nourriture; l'enceinte close comprend également au moins un compartiment inférieur destiné à recevoir les œufs des insectes contenus dans le compartiment supérieur; l'enceinte close comprend en outre au moins un élément de séparation desdits compartiments supérieur et inférieur , ledit élément de séparation comprenant des pores ; lesdits compartiments supérieur et inférieur sont amovibles et séparables de l'enceinte, indépendamment l'un de l'autre.

On connait aussi le brevet US10667502 décrivant une serre d'élevage à grande échelle et à plusieurs travées pour des mouches soldats noires adultes, comprenant des corps de serre. Chaque corps de serre comprend une chambre à pupes et une chambre d'accouplement et de ponte ; une paroi de séparation est prévue entre la salle des pupes et la salle d'accouplement et de ponte, la paroi de séparation est pourvue d'un trou de porte communiquant la salle des pupes et la salle d'accouplement et de ponte ; la chambre des pupes est opaque; le corps de serre comprend en outre un dispositif de guidage de lumière qui introduit la lumière du soleil dans la chambre d'accouplement et de ponte.

### Inconvénients de l'art antérieur

Ces solutions ne sont pas totalement satisfaisantes car l'introduction des récipients contenant les pupes, le retrait des œufs et les opérations de maintenance se font dans un espace dont les parois doivent empêcher la sortie des mouches hors de leur espace confiné. De façon plus générale, les solutions de l'art antérieur laissent subsister un risque excessif d'échappées de mouches hors des cages notamment lors de l'introduction ou de l'extraction des collecteurs d'œufs.

Par ailleurs, les solutions de l'art antérieur présentent une géométrie relativement complexe, avec des recoins pouvant piéger des mouches et rendant difficile le nettoyage complet.

Par ailleurs, les solutions de l'art antérieur présentent l'inconvénient de multiplier les risques de pontes hors collecteurs en raison de leurs géométries complexes présentant des recoins multiples. En effet, il est connu que les mouches ont tendance à pondre dans les recoins.

Enfin, les solutions de l'art antérieur ne sont pas optimales pour les interventions de nettoyage ou de maintenance en l'absence de mouches.

### Solution apportée par l'invention

Afin de remédier à ces inconvénients, la présente invention concerne selon son acception la plus générale une cage pour la production industrielle d'œufs d'insectes, conforme à la revendication 1.

Avantageusement, ladite zone sombre présente au moins une sortie latérale orientée vers une zone lumineuse de ladite cage.

Selon une variante, l'une desdites parois présente au moins une ouverture de section adaptée à la section transversale de moyens d'oviposition.

De préférence, les jonctions entre deux surfaces planes de ladite cage présentent une forme arrondie. En évitant les angles vifs et les recoins, on réduit le taux de ponte hors collecteurs. Ces jonctions concernent principalement l'assemblage des parois. Elles concernant aussi, optionnellement, le raccordement des accessoires sur lesdites parois.

L'invention concerne aussi une juxtaposition de cages conformes à au moins un des éléments ci-dessus.

### Description détaillée d'un exemple non limitatif de réalisation

D'autres caractéristiques et avantages ressortiront de la description qui va suivre de l'invention, description donnée à titre d'exemple uniquement, se référant aux dessins annexés sur lesquels :
- La figure 1 représente une vue en perspective d'une installation comportant des cages selon l'invention.
- La figure 2 représente une vue en perspective d'une ouverture bordée de moyens complémentaires de fermeture étanche, pour l'insertion et le retrait de collecteurs d'œufs, pour une cage selon l'invention.

### Principe général

L'invention concerne une installation d'élevage d'insectes dédiés à la production d'œufs, dans le cadre d'un système de production et de transformation industrielles d'insectes. De façon usuelle, une telle installation comprend une chambre de production d'œufs configurée pour recevoir des pupes d'insectes qui vont achever la nymphose dans la chambre, et permettre aux insectes adultes émergés de s'accoupler et de pondre des œufs sur des collecteurs généralement associés à un moyen d'oviposition pour éviter la dispersion des lieux de ponte. Des collecteurs, par exemple des panneaux nervurés, constituent les zones de ponte pour faciliter le regroupement et la collecte des œufs et leurs transports. Ces collecteurs sont ensuite déplacés dans une autre installation destinée à la maturation et à l'éclosion des œufs. Les insectes adultes sont souvent soumis à un éclairage contrôlé avec au moins une longueur d'onde de lumière propice à l'accouplement entre les insectes. Outre la longueur d'onde, l'intensité de l'éclairage est également contrôlée au cours de la journée pour être propice à l'accouplement en modulant cette intensité et en alternant les périodes avec et sans éclairage.

La simplicité des manipulations et du nettoyage constitue un enjeu essentiel pour permettre une production industrielle maîtrisée. L'installation comporte un groupement de cages (100,101) présentant un fond ouvert installées dans un bâtiment aux conditions climatiques contrôlées, vitré pour un éclairage naturel, ou opaque pour un éclairage artificiel. Le sol du bâtiment sert de fermeture inférieure des cages (100,101). Le sol du bâtiment peut présenter des moyens d'évacuation pour la collecte des déchets. A l'intérieur de ce bâtiment, seule la température est régulée : la valeur de cette température doit être la plus proche possible de la valeur à l'intérieur des cages tout en permettant des conditions de travail acceptables pour les opérateurs présents dans le bâtiment. Un deuxième système permet ensuite de contrôler plus finement les conditions climatiques (notamment l'humidité et la température) à l'intérieur des cages.

Chaque cage (100,101) est une construction légère destinée à définir un espace parallélépipédique de confinement des insectes évoluant dans la cage. Les cages (100) situées au cœur du groupement sont constituées d'une paroi avant (10) rigide, par exemple en Plexiglass (nom commercial), de deux parois latérales (40) communes à deux cages voisines, d'une paroi arrière (20) commune à deux cages voisines et d'un plafond (60). Les cages (101) situées aux extrémités du groupement sont constituées de deux parois latérales (30, 40).

Selon une réalisation particulière, la paroi (20) est constituée par un mélange de filet et de parois rigides : des parois rigides sont prévues en face des collecteurs pour pouvoir y fixer les dispositifs d'accrochage facilement, le reste sera du filet pour permettre la circulation d'air. La paroi (30) est rigide et étanche car elle délimite le groupe de cages.

De préférence, les cages forment une structure à fond ouvert, sans fond, qui sont déposées et fixées sur le sol d'un bâtiment équipé par exemple avec des moyens d'évacuation pour la collecte des déchets.

La paroi (40) est totalement en filet pour permettre la circulation d'air (à part une petite corniche arrondie en bas, en haut et sur les côtés pour éviter les angles).

La paroi avant (10) présente dans l'exemple décrit une ouverture (11) munie de deux portes (12, 13). Cette ouverture (11) débouche dans un sas d'émergence sombre et sans éclairage (14) formé à l'intérieur de la cage (100, 101) par exemple par des parois opaques. Au moins une des parois est perméable à l'air pour pouvoir mettre en conditions climatiques ce sas d'émergence. Ce sas (14) communique avec le reste de l'enceinte par des conduits (15) permettant aux mouches de rejoindre l'espace de reproduction et de ponte après émergence. La différence de luminosité résultant de l'opacité des parois constituant le sas (14) incite les mouches à quitter la zone de nymphose.

Les portes (12, 13) permettent d'ouvrir l'accès au sas (14) pour introduire au moins un récipient à pupes (16). L'obscurité régnant dans le sas (14) fait que peu de mouches sont présentes dans le sas (14), et les portes (12, 13) permettent d'introduire ou retirer un récipient à pupes (16) sans risque d'échappées. Optionnellement, un abaissement de la température permet de réduire l'activité des mouches et donc de minimiser le risque d'échappées.

La paroi avant (10) comporte également une ouverture (19), avantageusement pourvue de lèvres d'étanchéité élastiques, destiné à introduire un moyen d'oviposition, chargé par exemple de phéromones ou de substrat, qui peut être placé sous les collecteurs de ponte.

Ces collecteurs de ponte sont fixés sur un support solidaire des parois rigides avant (10) et arrière (20) et se présentent comme des plaques nervurées.

Des ouvertures (17, 18) également pourvues de barrières mécaniques empêchant toute échappées, ou de lèvres d'étanchéité élastiques, permettent d'introduire et d'extraire les collecteurs en empêchant les insectes de s'échapper de la cage pendant les opérations de manipulation des collecteurs.

Une porte (15) permet à un opérateur de s'introduire dans la cage notamment pour une intervention de dépannage ou de nettoyage.

De préférence, les cages sont juxtaposées pour former un groupement, typiquement entre 2 et 40 cages juxtaposées, de préférence entre 8 et 16 cages. Les avantages par rapport à des cages individuelles sont liés à la gestion des conditions climatiques : un groupe de cages permet en effet de réduire significativement les équipements CVC (Chauffage, Ventilation et Climatisation) puisque les conditions sont gérées à l'échelle du groupe de cages et non pas à l'échelle de la cage individuelle. Ce regroupement n'entraîne pour autant aucune perte de précision (tant que la taille du groupe reste raisonnable). Chaque cage dans un groupe de cages donné aura le même âge et aura ainsi besoin des mêmes conditions climatiques. Cela a aussi un avantage en termes de productivité puisque toutes les cages arriveront en fin de vie en même temps : l'étourdissement, le post-traitement des mouches et le nettoyage pourront se faire au niveau du groupe de cages.

### Exemple d'ouverture bordée de moyens complémentaires de fermeture étanche, pour l'insertion et le retrait de collecteurs d'œufs

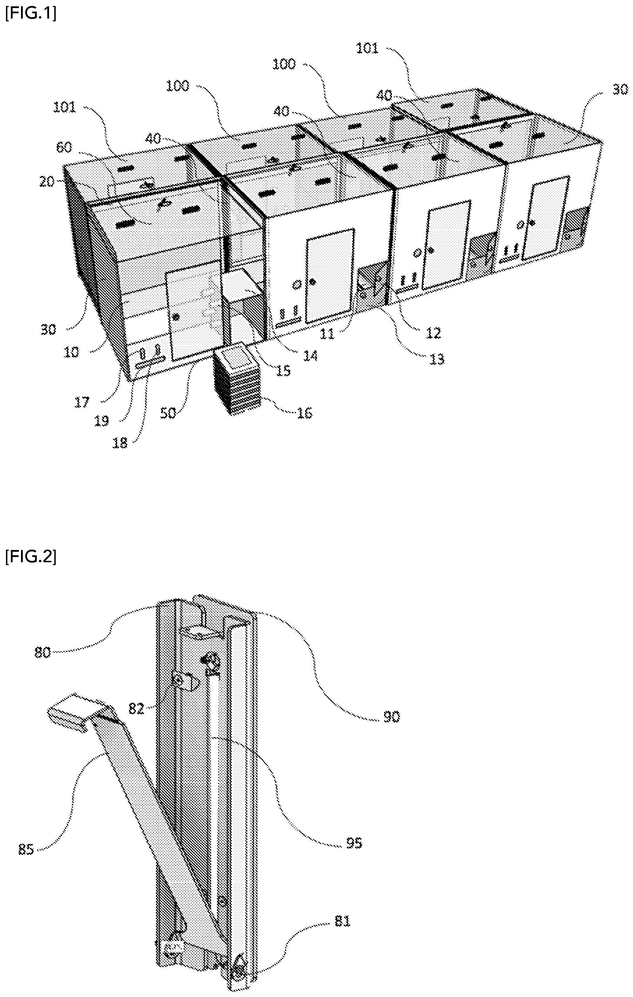

La figure 2 représente un exemple d'ouverture bordée de moyens complémentaires de fermeture étanche, pour l'insertion et le retrait de collecteurs d'œufs.

Elle est constituée par l'assemblage de deux tôles pliées (ou pièces de matière plastique) (80, 90) présentant chacune une fente (95) adaptée à l'insertion et à l'extraction d'un collecteur d'œufs, c'est-à-dire présentant une forme et des dimensions déterminées en fonction de la section transversale du collecteur d'œufs afin de permettre de le faire traverser la fente avec un jeu évitant que les mouches de la cage puissent s'échapper de la cage.

Cette fente (95) peut être masquée par une porte (85) basculante autour d'un axe transversal (81). Bien entendu, l'articulation peut aussi être prévue par rapport à un axe vertical. Une solution mécanique type élastique, aimant, loquet (82) assure le maintien de la porte (85) dans une position plaquée contre la fente (95).

Pour une paroi de cage souple formée par une toile ou un filet, les deux pièces (80 ; 90) sont positionnées de part et d'autre d'une découpe pratiquée dans la paroi, éventuellement gansée pour éviter la déchirure, et ensuite serrées l'une contre l'autres par des vis traversant les deux pièces (80, 90). La fente (95) pratiquée dans le filet peut être munie d'une fermeture à glissière de part et d'autre de la porte (85) pour permettre un accès additionnel.

Le montage est similaire pour des parois de cage rigides. On pratique dans ce cas une découpe d'une dimension supérieure à celle de la fente (95) et inférieure à la section des pièces (80, 90) afin de permettre de les positionner de part et d'autre de la paroi.

La tôle (80) peut être fixée par vissage par exemple sur l'extrémité frontale des rails de guidage des collecteurs d'œufs afin d'assurer le bon centrage.

## Revendications

1. - Cage pour la production industrielle d'œufs d'insectes, constituée par une enceinte délimitée par un plafond (60) et quatre parois latérales (10, 20, 30, 40) comprenant une paroi avant (10), définissant un espace confiné parallélépipédique pour les insectes, ladite enceinte contenant une zone de réception (14) d'au moins un récipient de pupes (16) et des moyens d'accrochage et d'extraction de collecteurs d'œufs pondus par les insectes confinés dans ladite cage (100), dans laquelle
- la paroi avant (10) de ladite enceinte présente :
∘ au moins une première ouverture (11) obturable par une première porte (12, 13) pour l'accès à ladite zone de réception (14) d'au moins un récipient de pupe (16),
- ladite cage présentant une zone de réception (14) constituant un espace interne sombre (14) communiquant d'une part avec ladite première porte (12, 13) et d'autre part avec le reste de ladite enceinte,
**caractérisée en ce que** la paroi avant (10) de ladite enceinte présente également :
∘ une deuxième porte d'accès à l'intérieur de la cage par un opérateur,
∘ au moins une ouverture (17, 18) bordée de moyens complémentaires de fermeture, pour l'insertion et le retrait de collecteurs d'œufs ;

2. - Cage selon la revendication 1 **caractérisé en ce que** ladite zone sombre (14) présente au moins une sortie latérale (15) orientée vers une zone lumineuse de ladite cage (10).

3. - Cage selon la revendication 1 **caractérisé en ce que** l'une desdites parois (10) présente au moins une ouverture (19) de section adaptée à la section transversale de moyens d'oviposition.

4. - Cage selon la revendication 1 **caractérisé en ce que** les jonctions entre deux surfaces planes de ladite cage présentent une forme arrondie.

5. - Ensemble pour la production industrielle d'œufs d'insectes, **caractérisé en ce qu'**il est constitué par une juxtaposition d'une pluralité de cages conformes à l'une au moins des revendications précédentes.

## Patentansprüche

1. Käfig für die industrielle Produktion von Insekteneiern, bestehend aus einem Gehäuse, das durch eine Decke (60) und vier Seitenwände (10, 20, 30, 40) begrenzt ist, umfassend eine Vorderwand (10), die einen eingeschlossenen quaderförmigen Raum für die Insekten definiert, wobei das Gehäuse einen Aufnahmebereich (14) für mindestens einen Puppenbehälter (16) und Mittel zum Einhängen und Herausnehmen von Sammelbehältern für die Eier, die von den in dem Käfig (100) eingeschlossenen Insekten gelegt wurden, enthält, wobei
- die Vorderwand (10) des Gehäuses Folgendes aufweist:
o mindestens eine erste Öffnung (11), die durch eine erste Tür (12, 13) verschließbar ist, um den Zugang zu dem Aufnahmebereich (14) für mindestens einen Puppenbehälter (16) zu ermöglichen,
- wobei der Käfig einen Aufnahmebereich (14) aufweist, der aus einem dunklen Innenraum (14) besteht, der einerseits mit der ersten Tür (12, 13) und andererseits mit dem Rest des Gehäuses in Verbindung steht,
**dadurch gekennzeichnet, dass** die Vorderwand (10) des Gehäuses weiter Folgendes aufweist:
o eine zweite Tür für den Zugang eines Bedieners in den Innenraum des Käfigs,
o mindestens eine Öffnung (17, 18), die von zusätzlichen Verschlussmitteln zum Einhängen und Herausnehmen von Sammelbehältern für die Eier umgeben ist;

2. Käfig nach Anspruch 1, **dadurch gekennzeichnet, dass** der dunkle Bereich (14) mindestens einen seitlichen Ausgang (15) aufweist, der zu einem beleuchteten Bereich des Käfigs (10) hin ausgerichtet ist.

3. Käfig nach Anspruch 1, **dadurch gekennzeichnet, dass** eine der Wände (10) mindestens eine Öffnung (19) aufweist, deren Querschnitt an den Querschnitt von Eiablagemitteln angepasst ist.

4. Käfig nach Anspruch 1, **dadurch gekennzeichnet, dass** die Verbindungsstellen zwischen zwei ebenen Flächen des Käfigs eine abgerundete Form aufweisen.

5. Anordnung zur industriellen Produktion von Insekteneiern, **dadurch gekennzeichnet, dass** sie aus einer Aneinanderreihung einer Vielzahl von Käfigen besteht, die mindestens einem der vorstehenden Ansprüche entsprechen.

## Claims

1. Cage for the industrial production of insect eggs, made up of an enclosure delimited by a ceiling (60) and four side walls (10, 20, 30, 40) comprising a front wall (10), defining a confined box-shaped space for the insects, said enclosure containing an area (14) for accommodating at least one container of pupae (16) and means for attaching and extracting collectors for collecting the eggs laid by the insects confined in said cage (100), in which
- the front wall (10) of said enclosure has:
∘ at least one first opening (11) that can be closed by a first door (12, 13) for accessing said area (14) for accommodating at least one container of pupae (16),
- said cage having an accommodating area (14) making up a dark inner space (14) that communicates, on the one hand, with said first door (12, 13), and on the other hand, with the rest of said enclosure,
**characterised in that** the front wall (10) of said enclosure also has:
∘ a second door for enabling an operator to access the inside of the cage,
∘ at least one opening (17, 18) lined with additional closure means, for inserting and removing the egg collectors.

2. Cage according to claim 1, **characterised in that** said dark area (14) has at least one side exit (15) oriented towards a light area of said cage (10).

3. Cage according to claim 1, **characterised in that** one of said walls (10) has at least one opening (19) with a section suitable for the cross-section of oviposition means.

4. Cage according to claim 1, **characterised in that** the junctions between two flat surfaces of said cage have a rounded shape.

5. Assembly for the industrial production of insect eggs, **characterised in that** it is made up by a juxtaposition of a plurality of cages, according to at least one of the preceding claims.
